# EUROPEAN PATENT APPLICATION

(11) **EP 2 163 276 A1**
(43) Date of publication of application: **17.03.2010**
(21) Application number: 08764888.7
(22) Date of filing: 30.05.2008
(51) Int. Cl.: A61M 25/01

(54) **GUIDE WIRE**

(30) Priority: 29.06.2007 JP 2007173306
(71) Applicant: Piolax Medical Devices, Inc., Yokohama-shi Kanagawa 240-0023 (JP)
(72) Inventor: TAKAHASHI, Hideo, Yokohama-shi Kanagawa 240-0023 (JP); YAZAKI, Yuta, Yokohama-shi Kanagawa 240-0023 (JP); MIYAMOTO, Toshiaki, Yokohama-shi Kanagawa 240-0023 (JP)
(74) Representative: Grünecker, Kinkeldey, Stockmair & Schwanhäusser Anwaltssozietät
(86) International application number: PCT/JP2008/059970
(87) International publication number: WO 2009/004876

(57) **Abstract**

There is provided a guide wire which can reduce sliding resistance against a tube such as a catheter to improve workability of introduction.

The guide wire 10 includes a core wire 20 which has a leading end portion 23 tapered, and concavo-convex irregularities 25 provided on an outer circumference of the core wire 20. The concavo-convex irregularities 25 are provided in a region with a length of at least 10% of the outer circumference of the core wire. When a catheter 5 is introduced into a tubular organ along an outer circumference of the guide wire 10, sliding resistance between the guide wire 10 and the catheter 5 can be reduced in order to move the catheter 5 smoothly because convex portions 37 of concavo-convex irregularities 35 of a resin film 30 formed in accordance with the concavo-convex irregularities 25 of the core wire 20 come into partial contact with an inner circumference of the catheter 5. Moreover, because the concavo-convex irregularities 25 are provided in the aforementioned range, sliding resistance between the guide wire 10 and the catheter 5 can be reduced in a wider range.

## Description

### Technical Field

The present invention relates to a guide wire used when inserting a tube such as a catheter into a human tubular organ such as a blood vessel, a ureter, a bile duct, a trachea, etc.

### Background Art

In recent years, for inspection/treatment in a human tubular organ such as a blood vessel, a ureter, a bile duct, a trachea, etc., a technique of inserting a catheter and injecting a medical agent such as a contrast medium or taking a part of a tissue by a pair of forceps or the like through the catheter has been performed. The catheter is inserted by the commonly-known Seldinger method. That is, after a puncture needle composed of an outer cylindrical needle and an inner cylindrical needle is introduced into a desired tubular organ through a skin, the inner cylindrical needle is pulled out of the outer cylindrical needle and a guide wire is inserted into the outer cylindrical needle so that a leading end portion of the guide wire reaches a target point in the tubular organ. Then, by pulling the outer cylindrical needle out of the tubular organ, the guide wire is left in the tubular organ. Then, a catheter is put on an outer circumference of a base end portion of the guide wire and moved along the outer circumference of the guide wire so that a leading end portion of the catheter reaches the target point.

As this type guide wire, a guide wire which has a guide wire body inserted into a channel of an endoscope and which is configured so that a leading end portion of the guide wire body is stopped while clamped between a guide wire fixing portion and a lever (forceps riser) which are provided in a leading end opening portion of the endoscope so that the lever can be opened and closed with respect to the guide wire fixing portion, has been heretofore disclosed, for example, in Patent Document 1 which will be described later. A fitting assisting portion is further provided in the leading end portion of the guide wire body so that the fitting assisting portion is fitted to a leading end of the guide wire fixing portion and a leading end of the lever when the guide wire body is clamped between the guide wire fixing portion and the lever. The fitting assisting portion is formed in the leading end portion of the guide wire body by convexly forming a spiral convex portion on or by winding a wire material like a coil on the outer circumference of the guide wire body.
Patent Document 1: JP-2001-340288-A

### Disclosure of the Invention

### Problem that the Invention is to Solve

The measure to coat the outer circumference of the guide wire with a hydrophilic resin, or the like, is taken so that slidability of the catheter along the guide wire can be improved when the catheter is introduced into a tubular organ along the outer circumference of the guide wire. However, a problem may occur in workability of introduction of the catheter because sliding resistance of the catheter against the guide wire cannot be reduced sufficiently. For example, when the catheter is introduced into a tubular organ having a lot of bent portions, the aforementioned problem occurs easily.

According to the aforementioned Patent Document 1, as an example of the fitting assisting portion, a spiral convex portion is provided in the leading end portion of the guide wire body. However, since the spiral convex portion is not provided for reducing sliding resistance against the catheter but formed only in a limited range of the leading end of the guide wire body, it is conceived that sliding resistance between the guide wire and the catheter cannot be reduced sufficiently so that workability of introduction of the catheter along the guide wire is hardly improved.

Therefore, an object of the invention is to provide a guide wire which can reduce sliding resistance against a tube such as a catheter, a puncture needle, a guide wire housing member, etc. to improve workability of introduction.

### Means for Solving the Problem

To achieve the foregoing object, a first aspect of the invention provides a guide wire **characterized in that:** the guide wire includes a core wire which has at least one end portion tapered, and concavo-convex irregularities provided on an outer circumference of the core wire; and the concavo-convex irregularities are provided in a range with a length of at least 10% of the outer circumference of the core wire.

According to the aforementioned invention, the concave-convex irregularities are provided on the outer circumference of the core wire. Accordingly, when a tube such as a catheter is introduced into a tubular organ along the outer circumference of the guide wire or when the guide wire is inserted into a tube such as a catheter, convex portions of the concavo-convex irregularities on the outer circumference of the core wire come into contact with an inner circumference of the tube so that a contact area of the outer circumference of the guide wire with the inner circumference of the tube can be reduced. As a result, when the tube such a catheter is introduced along the outer circumference of the guide wire or when the guide wire is inserted into the tube such as a catheter, sliding resistance between the guide wire and the tube can be reduced to move the guide wire and the tube smoothly.

Moreover, because the concavo-convex irregularities are provided in a region with a length of at least 10% of the outer circumference of the core wire, sliding resistance between the guide wire and the tube can be reduced in a wider range so that work of introduction can be made more smoothly when the tube is introduced deeply into the tubular organ along the outer circumference of the guide wire or when the guide wire is introduced into the tube which is long.

A second aspect of the invention provides the guide wire according to the first aspect of the invention, wherein the concavo-convex irregularities are provided at a pitch of 2-15/2.54cm.

According to the aforementioned invention, because the concavo-convex irregularities are provided at the aforementioned pitch, sliding resistance between the guide wire and the tube can be reduced more to improve workability of introduction.

A third aspect of the invention provides the guide wire according to the first or second aspect of the invention, wherein the concavo-convex irregularities have a height of 0.01-0.1mm.

According to the aforementioned invention, because the height of the concavo-convex irregularities is set in the aforementioned range, the outer diameter of the guide wire can be made as small as possible so that sliding resistance between the guide wire and the tube can be reduced more in a range to avoid spoiling of the stiffness of the guide wire.

A fourth aspect of the invention provides the guide wire according to any one of the first to third aspects of the invention, wherein the concavo-convex irregularities are formed by twisting a wire material forming the core wire.

According to the aforementioned invention, because the concavo-convex irregularities are formed by twisting a wire material forming the core wire, the concavo-convex irregularities can be formed by a relatively simple process. Moreover, the concavo-convex irregularities can be formed integrally with the outer circumference of the core wire by twisting the wire material and provided as a relatively gentle concavo-convex shape.

A fifth aspect of the invention provides the guide wire according to any one of the first to fourth aspects of the invention, wherein the guide wire further includes a resin film applied on the outer circumference of the core wire, and wherein concavo-convex irregularities are formed on an outer circumference of the resin film in accordance with the concavo-convex irregularities of the core wire.

According to the aforementioned invention, because the outer circumference of the resin film applied on the outer circumference of the core wire forms concavo-convex irregularities, a contact area between the guide wire and the tube can be reduced in the same manner as described above to thereby reduce sliding resistance. Moreover, because the resin film is provided as the outer circumference of the guide wire, frictional resistance against an inner circumference of the tube can be reduced to a relatively small value to thereby further improve slidability of the guide wire to the tube. In addition, because the resin film is applied on the outer circumference of the core wire having the concavo-convex irregularities provided thereon, the resin film is applied so as to enter into each concave portion between adjacent ones of convex portions formed in the core wire so that the resin film can be prevented from being displaced axially relative to the core wire.

### Effect of the Invention

According to the guide wire of the invention, the concavo-convex irregularities are provided on the outer circumference of the core wire. Accordingly, when a tube such as a catheter is introduced into a tubular organ along the outer circumference of the guide wire or when the guide wire is inserted into a tube such as a catheter, convex portions of the core wire come into contact with an inner circumference of the tube. Accordingly, a contact area of the outer circumference of the guide wire with the inner circumference of the tube can be reduced, so that sliding resistance between the guide wire and the tube can be reduced to move the guide wire and the tube smoothly.

Moreover, because the concavo-convex irregularities are provided in a region with a length of at least 10% of the outer circumference of the core wire, sliding resistance between the guide wire and the tube can be reduced in a wider range. Accordingly, when the tube is introduced deeply into the tubular organ along the outer circumference of the guide wire or when the guide wire is introduced into the tube which is long, work of introduction can be performed more smoothly.

### Best Mode for Carrying Out the Invention

An embodiment of a guide wire according to the invention will be described below with preference to the drawings.

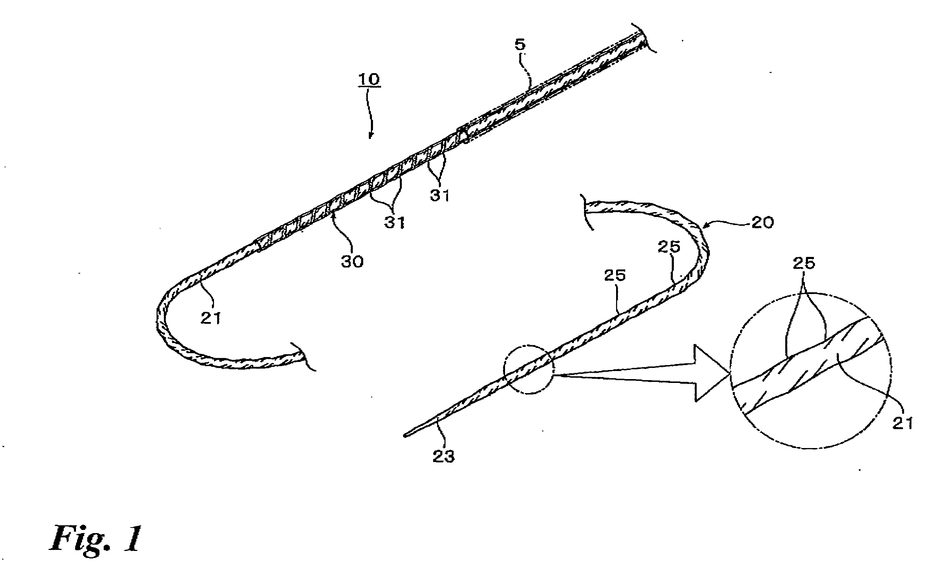

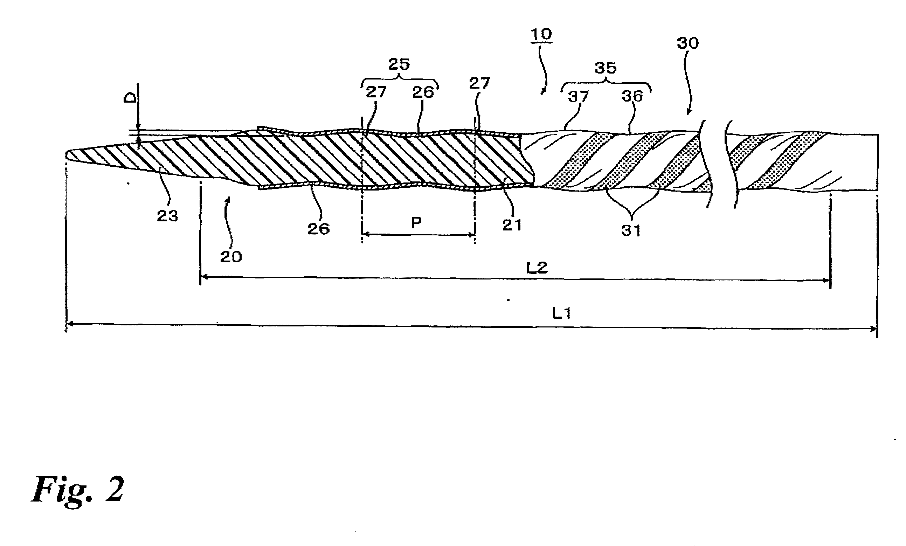

As shown in Figs. 1 and 2, the guide wire 10 in this embodiment includes a core wire 20 having a leading end portion 23 tapered, and a resin film 30 applied on an outer circumference of the core wire 20. The core wire 20 is made of a round wire extended with a predetermined length and has a circular sectional shape. The core wire 20 has a base portion 21, and the leading end portion 23 tapered gradually from the base portion 21 to a pointed tip. Although the length of the core wire 20 varies in accordance with a tubular organ as a target, a core wire 20 with a length L1 (see Fig. 2) of 1000-5500mm is used preferably. Although this embodiment shows the case where the leading end portion 23 of the core wire 20 is tapered, a base end portion opposite thereto may be tapered, that is, this embodiment can be applied to the case where at least one end portion is tapered.

The leading end portion 23 can be formed by machining, etching, etc. and its shape is not limited to the taper shape as shown in Figs. 1 and 2. For example, a step shape in which the diameter of the leading end portion 23 is reduced stepwise from the base portion 21 toward the pointed tip may be formed.

As shown in Fig. 2, the guide wire according to the invention is **characterized in that** concavo-convex irregularities 25 are provided in a region with a length L2 of at least 10% of the outer circumference of the core wire 20. That is, concavo-convex irregularities 25 are provided in a region with a length L2 of at least 10% of the length L1 of the core wire 20 and preferably in a range with a length L2 of 40%-98% (both inclusively) of the length L1 of the core wire 20. Referring also to Fig. 1, the aforementioned length L2 in this embodiment is set as a length of from the base end side of the leading end portion 23 of the core wire 20 to the base end side of the base portion 21, and the concavo-convex irregularities 25 are provided in the leading end portion 23 and the base portion 21 of the core wire 20. Moreover, in this embodiment, because the resin film 30 is applied on the outer circumference of the core wire 20 having the concavo-convex irregularities 25 provided thereon, concavo-convex irregularities 35 corresponding to the shape of the concavo-convex irregularities 25 of the core wire 20 are formed on an outer circumference of the resin film 30 (as will be described later).

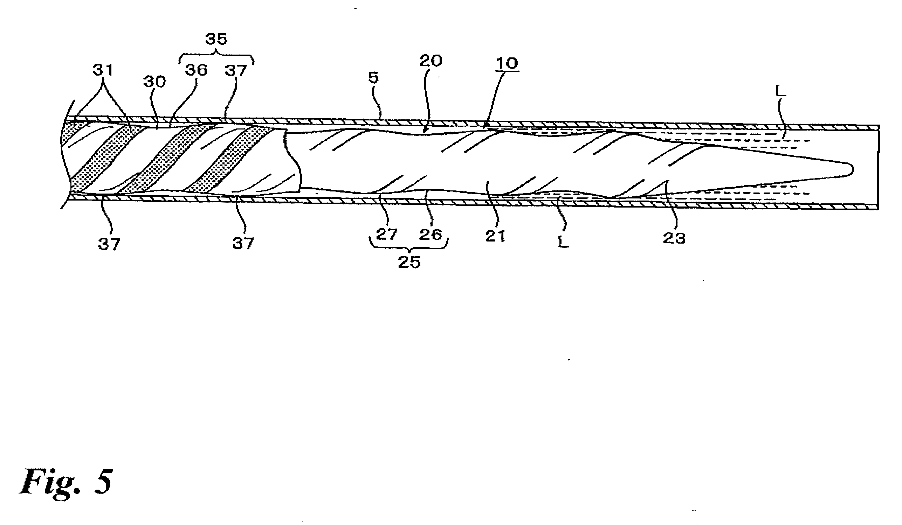

Because the concavo-convex irregularities 25 are provided in a predetermined region in this manner, a contact area between the guide wire 10 and a tube such as a catheter 5 shown in Fig. 5 can be reduced to thereby reduce sliding resistance between the tube such as a catheter and the guide wire 10. Incidentally, if the length L2 in which the concavo-convex irregularities 25 are formed is smaller than 10% of the length L1 of the core wire 20, it is undesirably impossible to reduce the contact area between the guide wire 10 and the tube sufficiently.

A radiopaque material made of one member selected from the group consisting of superelastic alloys such as an Ni-Ti alloy, an Ni-Ti-X (X=Fe, Cu, V, Co, etc.) alloy and a Cu-Zn-X (X=Al, Fe, etc.) alloy, stainless steel, piano wire materials, and metals such as Pt, Ti, Pd, Rh, Au and W and alloys of these metals is preferably used as the material of the core wire 20.

In this embodiment, the concavo-convex irregularities 25 provided in the core wire 20 are formed by twisting a wire material made of the aforementioned material. For example, the concavo-convex irregularities 25 are formed such that one end portion of a wire material made of the aforementioned material and has a circular sectional shape is fixed while chucked by clamps or the like, the other end portion of the wire material is held by a rotating device, and the other end portion of the wire material in this condition is rotated by a predetermined amount to thereby twist the wire material. Alternatively, the concavo-convex irregularities 25 may be formed such that opposite end portions of a wire material are held by a rotating device and one of the end portions of the wire material is rotated while the other end portion of the wire material is rotated in a rotating direction opposite to the one end portion to thereby twist the wire material. Incidentally, when the core wire 20 having the concavo-convex irregularities 25 is formed by twisting a wire material, it is preferable that the aforementioned processing of the leading end portion 23 of the core wire 20 is performed after the wire material twisting process.

As described above, in this embodiment, because the concavo-convex irregularities 25 are formed by twisting the wire material, the concavo-convex irregularities 25 can be formed by the aforementioned relatively simple process. Moreover, by twisting the wire material, the concavo-convex irregularities 25 can be formed integrally with the outer circumference of the core wire 20 and provided as a relatively gentle concavo-convex shape.

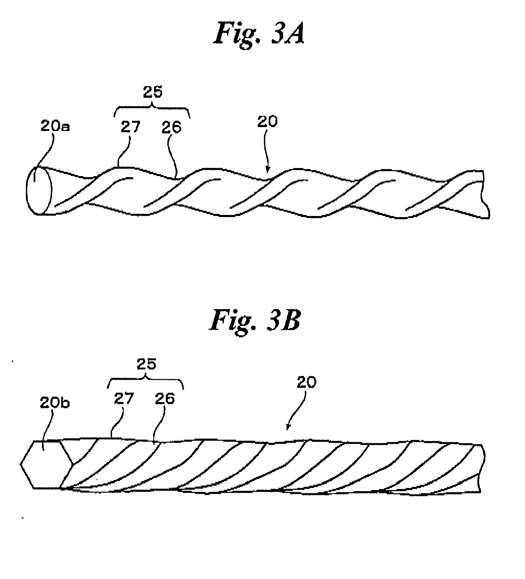

Although this embodiment shows the case where the concavo-convex irregularities 25 are formed by twisting a wire material having a circular sectional shape, this embodiment is not particularly limited thereto. The concavo-convex irregularities 25 may be formed by twisting a wire material 20a having an ellipse sectional shape as shown in Fig. 3A or may be formed by twisting a wire material 20b having a polygon (regular hexagon in the drawing) sectional shape as shown in Fig. 3B. Alternatively, the concavo-convex irregularities 25 may be formed by twisting a wire material having a different diameter. Incidentally, in the case where a wire material 20a or 20b having an ellipse or a polygon sectional shape is twisted as shown in Figs 3A and 3B, the vertical level difference of the concavo-convex shape can be distinct compared with the case where a wire material having a circular sectional shape is twisted.

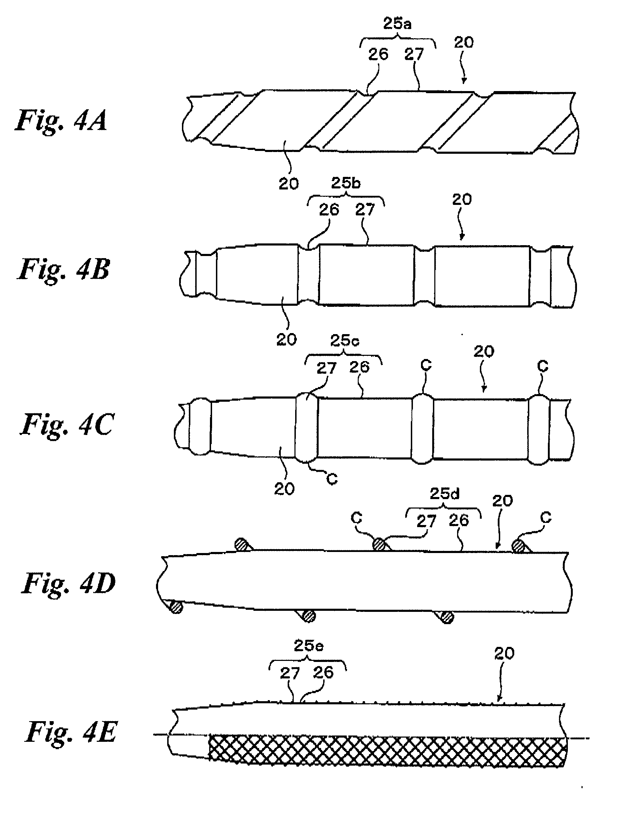

The aforementioned concavo-convex shape is not limited to the shape formed by twisting a wire material and concavo-convex shapes shown in Figs. 4A to 4E may be used. For example, as shown in Fig. 4A, concavo-convex irregularities 25a may be formed such that a spiral concave groove is formed along an axial direction in the outer circumference of the core wire 20 by machining, etching or the like so that this concave groove is provided as concave portions 26 while the outer circumferential portion of the core wire 20 where the concave portions 26 are not formed is provided as convex portions 27. Alternatively, as shown in Fig. 4B, concavo-convex irregularities 25b may be formed such that annular concave grooves are formed at regular intervals along an axial direction in the outer circumference of the core wire 20 so that these annular concave grooves are provided as concave portions 26 while the outer circumferential portion of the core wire 20 where the concave portions 26 are not formed is provided as convex portions 27. Incidentally, the annular concave grooves can be formed by machining, etching or the like, similarly to the spiral concave groove shown in Fig. 4A.

Further alternatively, as shown in Fig. 4C, concavo-convex irregularities 25c may be formed such that annular ring members are provided at regular intervals along an axial direction on the outer circumference of the core wire 20 and stuck to the outer circumference of the core wire 20 by an adhesive agent or the like so that the ring members are provided as convex portions 27 while the outer circumferential portion of the core wire 20 where the ring members are not stuck is provided as concave portions 26. Alternatively, as shown in Fig. 4D, concavo-convex irregularities 25d may be formed such that a coil made of a wire material wound at intervals of a predetermined pitch is fixed onto the outer circumference of the core wire 20 by an adhesive agent or the like. In this case, the coiled wire material is provided as convex portions 27 while the outer circumferential portion of the core wire 20 where the coiled wire material is not formed is provided as concave portions 26. Although Fig. 4D shows the case where a round wire having a circular sectional shape is used as the wire material for forming the coil, a flat wire having a rectangular sectional shape may be used. Further alternatively, as shown in Fig. 4E, concavo-convex irregularities 25e may be formed such that a mesh tube made of tube-shaped meshes by knitting and/or braiding wire materials is mounted on the outer circumference of the core wire 20. In the case of the mesh tube, in the same manner as in Fig. 4D, the wire materials are provided as convex portions 27 while the outer circumferential portion of the core wire 20 where the wire materials of the mesh tube are not mounted is provided as concave portions 26.

In the case of the concave-convex irregularities 25 shown in each of Figs. 4C to 4E, the annular ring member, the wire material of the coil or the wire material of the mesh tube for forming the convex portions 27 is preferably processed so that corner portions C of the convex portions 27 are rounded like R as shown in Fig. 4C or Fig. 4D. By rounding the corner portions C of the convex portions 27 in this manner, sliding resistance between the tube such as a catheter and the guide wire 10 can be reduced more.

The aforementioned concavo-convex irregularities 25 are provided so that the concave portions 26 and the convex portions 27 continue alternately in an axial direction of the core wire 20. The pitch of the concavo-convex irregularities 25 is preferably 2-15/2.54cm (1 inch). As shown in Fig. 2, one pitch P means the distance between the center of a convex portion 27 (the most protruding position of a convex portion 27, the same definition applies hereinafter) and the center of an adjacent convex portion 27 with interposition of a concave portion 26 from the convex portion 27. By providing the concavo-convex irregularities 25 at intervals of the aforementioned pitch, sliding resistance between the guide wire 10 and the tube can be reduced more to improve workability of introduction. Incidentally, when the pitch of the concavo-convex irregularities 25 is larger than 15/2.54cm so that the concavo-convex irregularities 25 are formed finely in the core wire 20 (that is, when the number of the concavo-convex irregularities 25 formed is large), the contact area with the inner circumference of the tube increases to make it undesirably difficult to reduce sliding resistance. On the other hand, when the pitch of the concavo-convex irregularities 25 is smaller than 2/2.54cm so that the concavo-convex irregularities 25 are formed roughly in the core wire 20 (that is, when the number of the concavo-convex irregularities 25 is small), there occurs a possibility that the inner circumference of the tube will come into contact with a concave portion 26 between convex portions 27 adjacent to each other when the tube such as a catheter is moved relative to the guide wire 10. Also in this case, reduction in sliding resistance is hardly attained undesirably.

Moreover, as shown in Fig. 2, the height D of the concavo-convex irregularities 25 (that is, the distance from the most protruding portion of each convex portion 27 to the most depressed portion of each concave portion 26) is preferably 0.01-0.1mm. By setting the height D of the concavo-convex irregularities 25 in the aforementioned range, the outer diameter of the guide wire 10 can be made as small as possible so that sliding resistance between the guide wire 10 and the tube such as a catheter can be reduced more in a range to avoid spoiling of the stiffness of the guide wire 10. Incidentally, if the height D of the concavo-convex irregularities 25 is smaller than 0.01mm, the guide wire 10 frequently comes into contact with the inner circumference of the tube so that the effect of reducing sliding resistance between the guide wire 10 and the tube runs short undesirably. On the other hand, if the height D of the concavo-convex irregularities 25 is larger than 0.1mm, the outer diameter of the guide wire 10 becomes large enough to spoil its own flexibility to thereby obstruct workability of introduction into the tubular organ undesirably.

The outer circumference of the core wire 20 having the concavo-convex irregularities 25 formed therein as described above is designed to be coated with the resin film 30. The resin film 30 is shaped like a tube. For example, a fluorocarbon resin such as polytetrafluoroethylene (PTFE), perfluoroalkoxy resin (PFA), ethylene tetrafluoride-propylene hexafluoride copolymer (FEP), ethylene tetrafluoride-ethylene copolymer (ETFE), etc., or a synthetic resin such as polyurethane, Nylon elastomer, polyether block amide, polyethylene, polyvinyl chloride, vinyl acetate, etc. is preferably used as the material of the resin film 30. Powder of BaSO₄, Bi, W, or the like, may be contained in the aforementioned resin material to make the resin material radiopaque. In addition, the outer circumference of the tube-like resin film 30 is preferably coated with a hydrophilic resin such as polyvinylpyrrolidone, polyethylene glycol, methyl vinyl ether-maleic anhydride copolymer, etc. in order to give slidability and thrombus deposition prevention.

Moreover, a spiral pattern 31 of a different color from the base color of the resin film 30 is provided on the resin film 30 along an axial direction. Because the resin film 30 is color-code with plural colors, visibility can be improved when the guide wire 10 is operated by a fiberscope or the like while an image of the guide wire 10 is displayed on a monitor.

For example, a heat-shrinkable resin tube is used as the aforementioned resin film 30. After the heat-shrinkable resin tube is put on the outer circumference of the core wire 20, the heat-shrinkable resin tube is heat-shrunken so as to be fixed onto the outer circumference of the core wire 20.

On this occasion, because the concavo-convex irregularities 25 are provided in the core wire 20 as described above, the resin film 30 adheres to the outer circumference of the core wire 20 along the shape of the concavo-convex irregularities 25. As a result, concavo-convex irregularities 35 composed of concave portions 36 and convex portions 37 are formed in the outer circumference of the resin film 30. Because the outer circumference of the core wire 20 with the concavo-convex irregularities 25 provided thereon is coated with the resin film 30 in this manner, the resin film 30 is applied so as to enter into each concave portion 26 between adjacent ones of the convex portions 27 of the concavo-convex irregularities 25 formed in the core wire 20 so that the resin film 30 can be prevented from being displaced axially relative to the core wire 20.

Usage of the guide wire 10 of the invention configured as described above will be described next. The guide wire 10 is used when a catheter 5 is guided to a target point of a tubular organ not shown, for example, in the Seldinger method which is commonly known. That is, after a puncture needle (not shown) composed of an outer cylindrical needle and an inner cylindrical needle is introduced into a desired tubular organ through a skin, the inner cylindrical needle is pulled out of the outer cylindrical needle and then the guide wire 10 is inserted into the outer cylindrical needle so that a leading end portion of the guide wire 10 reaches a target point of the tubular organ. After the outer cylindrical needle is then pulled out of the tubular organ, the catheter 5 is put on the outer circumference of the base end portion of the guide wire 10 and moved along the outer circumference of the guide wire 10 (see Fig. 5), so that a leading end portion of the catheter 5 can reach the target point. Incidentally, the catheter 5 may be moved by repetition of such an operation that the catheter 5 is moved following the guide wire 10 when the leading end of the guide wire 10 is moved a little.

On this occasion, because the convex portions 37 of the concavo-convex irregularities 35 formed in the resin film 30 in accordance with the concavo-convex irregularities 25 provided in the outer circumference of the core wire 20 in the guide wire 10 come into partial contact with the inner circumference of the catheter 5 as shown in Fig. 5, the contact area of the outer circumference of the guide wire 10 with the inner circumference of the catheter 5 can be reduced. As a result, when the catheter 5 is introduced along the outer circumference of the guide wire 10, sliding resistance between the guide wire 10 and the catheter 5 tube can be reduced to improve slidability of the catheter 5 to move the catheter 5 smoothly.

Moreover, because the concavo-convex irregularities 25 are provided in a region with a length L2 of at least 10% of the outer circumference of the core wire 20, sliding resistance between the guide wire 10 and the catheter 5 can be reduced in a wider range so that work of introduction can be performed more smoothly when the catheter 5 is introduced deeply into the tubular organ along the outer circumference of the guide wire 10.

Incidentally, the aforementioned effect can be obtained not only when the catheter 5 is moved along the guide wire 10 but also when the guide wire 10 is inserted into a tube such as a rumen of an endoscope, an outer cylindrical needle of a puncture needle, a sheath, etc. or when the guide wire 10 is inserted into a tube-like guide wire housing member to house and hold the guide wire 10 during conveyance of the guide wire 10. Sliding resistance between the tube and the guide wire 10 can be reduced to move the guide wire 10 smoothly.

The tapered leading end portion 23 of the guide wire 10 hardly comes into contact with the inner circumference of the tube. However, when the tube is bent, the leading end portion 23 may come into contact with the inner circumference of the tube. Contrary, in this embodiment, even when the tube is bent, sliding resistance in the bent portion can be reduced because the concavo-convex irregularities 25 are provided also in the leading end portion 23 of the core wire 20.

As shown in Fig. 5, a liquid L such as a physiological saline solution, a contrast medium, a blood, etc. may remain in a gap between the outer circumference of the guide wire 10 and the inner circumference of the catheter 5. According to this guide wire 10, the liquid L can be discharged to the outside of the catheter 5 so as to be scraped off by the concavo-convex irregularities 35 provided in the outer circumference of the guide wire 10, so that predetermined work such as radiographic work can be performed steadily. Moreover, because resistance due to the liquid L remaining in the catheter 5 can be reduced, workability of introduction of the catheter 5 along the guide wire 10 can be improved more.

Moreover, in this embodiment, the outer circumference of the core wire 20 with the concavo-convex irregularities 25 provided thereon is coated with the resin film 30 so that the outer circumference of the guide wire 10 is composed of the resin film 30 which is softer than the core wire 20. Accordingly, frictional resistance to the inner circumference of the tube can be reduced to a relatively small value compared with the case where the core wire 20 comes into direct contact with the inner circumference of the tube, so that slidability of the guide wire 10 to the tube can be improved. Moreover, the concavo-convex irregularities 35 are not directly provided in the resin film 30 but indirectly provided in the outer circumference of the resin film 30 by adhering the resin film 30 to the concavo-convex irregularities 25 provided in the outer circumference of the core wire 20. Accordingly, the concavo-convex irregularities 35 can be provided evenly along the outer circumference of the resin film 30 without crushing and deformation of the concavo-convex irregularities 35.

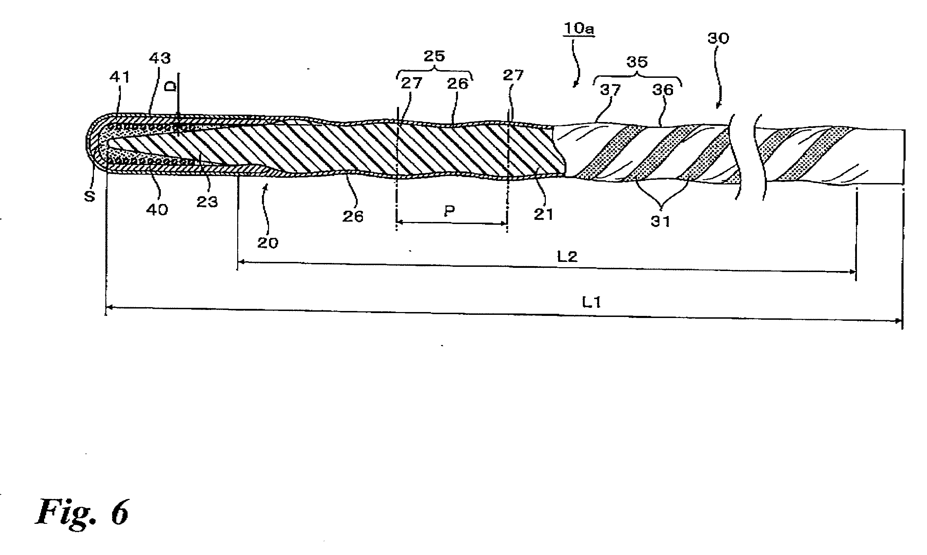

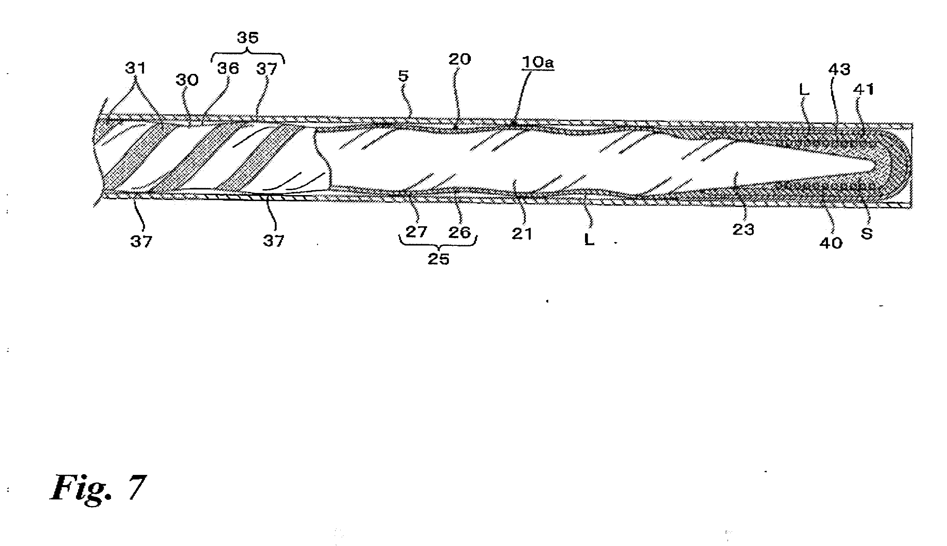

Figs. 6 and 7 show another embodiment of the guide wire according to the invention. Incidentally, parts substantially the same as those in the aforementioned embodiment are referred to by the same numerals and signs and description thereof will be omitted.

The guide wire 10a according to this embodiment is different from that according to the aforementioned embodiment in that a coil 40 is mounted on the leading end portion 23. That is, as shown in Fig. 6, a coil 40 is mounted on the outer circumference of the tapered leading end portion 23 of the core wire 20 through an adhesive agent S such as a cyanoacrylate resin. From the viewpoint of making the coil 40 serve as a marker at radiography, a metal such as Au, Pt, Ag, Bi or W or an alloy containing at least one of these metals is preferably used as the coil 40. A bottomed tube 41 of a synthetic resin such as polyurethane is further stuck onto the outer circumference of the coil 40 by an adhesive agent or in a heat-shrinking manner. An outer circumference of a base end portion of the tube 41 is covered with the outer circumference of the leading end portion of the resin film 30 applied on the outer circumference of the base portion 21 of the core wire 20. The outer circumference of the tube 41 is further coated with a hydrophilic resin film 43 and the base end portion of the hydrophilic resin film 43 strikes against the leading end surface of the resin film 30 so that there is no difference in level of the boundary surface between the hydrophilic resin film 43 and the resin film 30.

In the guide wire 10a configured as described above, like the guide wire 10 according to the aforementioned embodiment, as shown in Fig. 7, when the catheter 5 is moved along the outer circumference of the guide wire 10a, the convex portions 37 of the concavo-convex irregularities 35 of the resin film 30 come into partial contact with the inner circumference of the catheter 5 to thereby reduce sliding resistance between the guide wire 10a and the catheter 5 to make it possible to move the catheter 5 smoothly. When the coil 40 mounted on the leading end portion of the guide wire 10a is made radiopaque, the guide wire 10a can be introduced into the tubular organ while the position of the leading end portion of the guide wire 10a can be visually recognized. In addition, the guide wire 10a is softened by the coil 40 so that the tubular organ can be prevented from being damaged by the guide wire 10a.

### Brief Description of the Drawings

[Fig. 1] A perspective view showing an embodiment of a guide wire according to the invention.
[Fig. 2] A partly sectionalized side view of the guide wire. [Figs. 3A and 3B] Views showing the case where different-shape core wires for forming the guide wire are twisted, Fig. 3A being an explanatory view in the case where a core wire having an ellipse sectional shape is twisted, Fig. 3B being an explanatory view in the case where a core wire having a polygon sectional shape is twisted.
[Figs. 4A to 4E] Views showing other shapes of concavo-convex irregularities of the guide wire, Fig. 4A being an explanatory view showing concavo-convex irregularities formed from a spiral concave groove, Fig. 4B being an explanatory view showing concavo-convex irregularities formed from annular concave grooves, Fig. 4C being an explanatory view showing concavo-convex irregularities formed from annular ring members, Fig. 4D being an explanatory view showing concavo-convex irregularities formed from a coil, Fig. 4E being an explanatory view showing concavo-convex irregularities formed from a mesh member.
[Fig. 5] An explanatory view showing a state in use of the guide wire.
[Fig. 6] A partly sectionalized side view, showing another embodiment of the guide wire according to the invention.
[Fig. 7] An explanatory view showing a state in use of the guide wire.

### Description of Reference Numerals and Signs

- 10, 10a: guide wire
- 20, 20a,: 20b core wire
- 23: leading end portion
- 25, 25a,: 25b, 25c, 25d concavo-convex irregularities
- 30: resin film

## Claims

1. A guide wire **characterized in that:**
the guide wire comprises a core wire which has at least one end portion tapered, and concavo-convex irregularities provided on an outer circumference of the core wire; and
the concavo-convex irregularities are provided in a range with a length of at least 10% of the outer circumference of the core wire.

2. The guide wire of Claim 1,
wherein the concavo-convex irregularities are provided at a pitch of 2-15/2.54cm.

3. The guide wire of Claim 1 or 2,
wherein the concavo-convex irregularities have a height of 0.01-0.1mm.

4. The guide wire of any one of Claims 1 to 3,
wherein the concavo-convex irregularities are formed by twisting a wire material forming the core wire.

5. The guide wire of any one of Claims 1 to 4,
wherein the guide wire further comprises a resin film applied on the outer circumference of the core wire, and
wherein concavo-convex irregularities are formed on an outer circumference of the resin film in accordance with the concavo-convex irregularities of the core wire.
